# EUROPÄISCHE PATENTSCHRIFT

(11) **EP 1 928 813 B1**
(45) Veröffentlichungstag und Bekanntmachung des Hinweises auf die Patenterteilung: **10.10.2012**
(21) Anmeldenummer: 06792687.3
(22) Anmeldetag: 03.08.2006
(51) Int. Cl.: C07C 45/40, C07C 47/02, C07C 47/54, C07C 47/42, C07C 49/185

(54) **DARSTELLUNG VON ALDEHYDEN DURCH OZONOLYSE SEKUNDÄRER ALLYLALKOHOLE**
SYNTHESIS OF ALDEHYDES BY OZONOLYSIS OF SECONDARY ALLYL ALCOHOLS
PREPARATION D'ALDEHYDES PAR OZONOLYSE D'ALCOOLS D'ALLYLE SECONDAIRES

(30) Priorität: 28.09.2005 DE 102005046535
(43) Veröffentlichungstag der Anmeldung: 11.06.2008
(73) Patentinhaber: Symrise AG, 37603 Holzminden (DE)
(72) Erfinder: NOBIS, Markus, CH-3250 Lyss (CH); HÖLSCHER, Bernd, 37620 Halle (DE)
(74) Vertreter: Eisenführ, Speiser & Partner
(86) Internationale Anmeldenummer: PCT/EP2006/065053
(87) Internationale Veröffentlichungsnummer: WO 2007/036381

(56) Entgegenhaltungen:
- EP-A- 1 354 865
- US-A- 3 664 810
- EVEREST, DIANA J.; GRANT, PETER K.; SLIM, GEORGE C.; YEO, IRENE K. L.: "A Mechanism for Anomalous Ozonolysis" AUSTRALIAN JOURNAL OF CHEMISTRY., Bd. 41, 1988, Seiten 1025-1035, XP008070795
- THORSTEN HASSELSTRÖM: "Über die Ozonisation des Sabinols" CHEMISCHES ZENTRALBLATT, 1927, Seiten 2295-2296, XP008070747
- P. A. LEVENE, A. WALTI: "Polymerization of the alpha-Hydroxyaldehydes" JOURNAL OF BIOLOGICAL CHEMISTRY., Bd. 94, 1931, Seiten 353-360, XP002406188 USAMERICAN SOCIETY OF BIOLOCHEMICAL BIOLOGISTS, BIRMINGHAM, AL.
- SIEGFRIED HUNECK ET AL.: "New Labdane Diterpenoids from the Liverwort" JOURNAL OF CHEMICAL RESEARCH. MINIPRINT., Bd. 5, 1986, Seiten 1601-1656, XP008070767 GBSCIENTIFIC REVIEWS, NORTHWOOD

## Beschreibung

Die Erfindung betrifft ein Verfahren zur Darstellung von Aldehyden aus sekundären Alkoholen mit einer alpha-ständigen Doppelbindung (im Folgenden auch: sekundäre Allylalkohole) mittels Ozonolyse.

Tertiäre Alkohole mit einer alpha-ständigen Doppelbindung werden herkömmlicherweise in Gegenwart anorganischer Oxidationsmittel (z.B. KMnO₄, OsO₄, H₂SO₄/H₂CrO₄) in die entsprechenden Carbonylverbindungen überführt. Insbesondere ist bekannt, einen Alkohol der Formel (I) auf diese Weise in ein Keton der Formel (II) zu überführen: 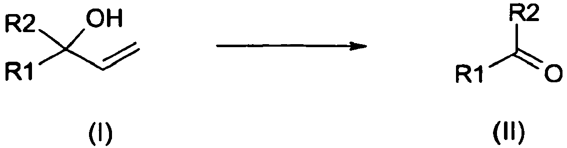 wobei die Reste R1, R2 unabhängig voneinander Alkyl, Alkenyl, Alkinyl, Cycloalkyl oder Aryl sein können. Die Reste können dabei verzweigt oder unverzweigt, substituiert oder unsubstituiert sowie die Arylreste ferner kondensiert sein. Alkohole der Formel (I) werden teilweise aus natürlichen Quellen bezogen, sind jedoch auch auf synthetischem Wege zugänglich.

Problematisch hingegen erweist sich die Darstellung von Aldehyden aus sekundären Alkoholen mit einer alpha-ständigen Doppelbindung (R2 = H in Formel (I)) auf diesem Weg. Aufgrund des Reaktionsmechanismus werden Nebenprodukte durch die verschiedenen Möglichkeiten zur Fragmentierung der Intermediate gebildet, wie im folgenden Schema dargestellt:

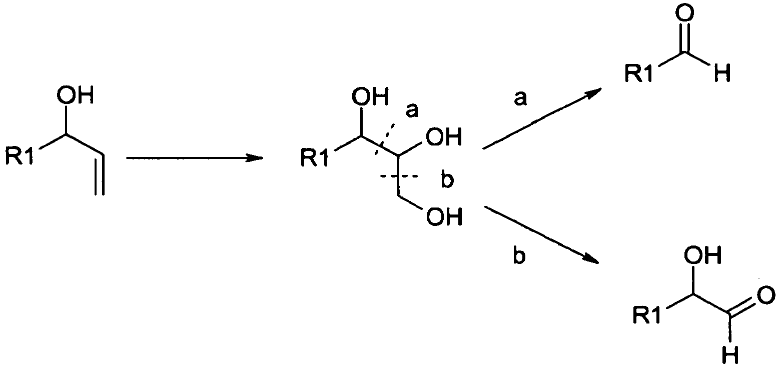

Aldehyde stellen für verschiedene Bereiche der chemischen Industrie wichtige (Zwischen)Produkte dar. Die Darstellung von Aldehyden ist beispielsweise durch die Umsetzung von olefinischen Verbindungen mit einem Gemisch aus Kohlenmonoxid und Wasserstoff an Metallkatalysatoren (Hydroformylierung), durch die Reaktion von Aromaten mit Dimethylformamid (sog. Vilsmeier-Haak-Synthese), oder auch durch die Umsetzung von Ketonen nach Darzens möglich.

Die genannten Darstellungsmethoden zeichnen sich durch die Verwendung von metallhaltigen Reaktanden oder durch die Verwendung giftiger Reagenzien aus. Weiterhin sind die jeweiligen Methoden auf die jeweilige Substratklasse und das vorliegen bestimmter Eigenschaften im Molekül begrenzt.

In der Literatur sind verschiedene Beispiele bekannt, in denen durch den ozonolytischen Abbau von tertiären Allylalkoholen Ketone gebildet werden. WO 91/09852 beschreibt beispielweise ein zweistufiges Verfahren zur Herstellung von Sclareolid (auch (-)-Norlabdanoxid) aus Sclareol, bei dem in erster Stufe ein oxidativer Abbau von Sclareol in Gegenwart von Rutheniumsalzen oder Kaliumpermanganat und in einer zweiten Stufe das gebildete Zwischenprodukt mit der Persäure und/oder Persäuresalzen zum Sclareolid oxidiert wird.

Die in herkömmlichen Verfahren genutzten Oxidationsmittel sind aufgrund ihrer Giftigkeit für Mensch und Umwelt sowie ihrer dadurch erschwerten Handhabbarkeit nachteilig. Dieser Nachteil erschwert insbesondere die industrielle Umsetzung von sekundären Alkoholen mit einer alpha-ständigen Doppelbindung.

Es ist deshalb versucht worden, diese Verfahren abzuändern und insbesondere neue Oxidationsmittel zu verwenden. Am Beispiel der Umsetzung von Sclareol ist in EP 0 822 191 A1 und Fekih et al. (J. Soc. Chim. Tunisie, 2001, 4(9), 909) das jeweils zweistufige Verfahren zur Darstellung von Sclareoloxid (VIIb) aus dem tertiären Allylalkohol Sclareol (VI) durch Ozonolyse beschrieben worden:

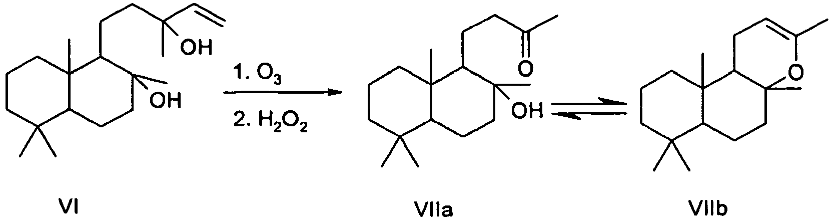

In einer ersten Stufe wird die Allylalkohol-Gruppe des Sclareols durch Addition von Ozon zum entsprechenden Ozonid umgesetzt. In einer zweiten Stufe wird anschließend das Ozonid durch Aufarbeitung mit alkalischem H₂O₂ in das gewünschte Sclareoloxid überführt. Die Reaktion kann in verschiedenen organischen Lösungsmitteln, wie Dichlormethan, Methanol oder Ethanol durchgeführt werden.

Bei der Umsetzung fallen jedoch im ersten Schritt große Mengen des stark reaktiven Ozonids an, so dass erhebliche Sicherheitsmaßnahmen für das Durchführen der Reaktion erforderlich sind. Insbesondere wird eine leistungsfähige Kühlung benötigt, um die Reaktion sicher durchführen zu können. Diese Nachteile fallen besonders bei einer industriellen Umsetzung stark ins Gewicht.

In EP 1 354 865 A1 ist ein bestimmtes Verfahren zur Herstellung spezieller aromatischer Aldehyde durch Ozonolyse von aromatischen Alkenen beschrieben. Diana J. Everest et al. beschreiben in der Publikation "A Mechanism for Anomalous Ozonolysis" (Aust. J. Chem., 1988, 41, 1025-35) einen Mechanismus und spezielle Produkte anomaler Ozonolyse.

Der Erfindung liegt die Aufgabe zugrunde, ein Verfahren zum Darstellen eines Aldehyds aus einem sekundären Alkohol mit einer alpha-ständigen Doppelbindung anzugeben, das die oben angegebenen Nachteile herkömmlicher Oxidationsverfahren begrenzt oder diese ganz vermeidet. Das Verfahren sollte insbesondere ohne die bisher erforderlichen starken Sicherheitsmaßnahmen durchführbar sein. Ebenfalls soll der Gebrauch von metallhaltigen und/oder giftigen Oxidationsmitteln vermieden und die Bildung von Aldehydgruppen ermöglicht werden, ohne dass eine Weiteroxidation zu den korrespondierenden Carbonsäuren in nennenswertem Ausmaß erfolgt.

Die Aufgabe wird gelöst durch ein Verfahren zur Darstellung eines Aldehyds aus einem sekundären Allylalkohol, umfassend die Schritte:
a) Bereitstellen des Alkohols,
b) Behandeln des Alkohols mit Ozon in Anwesenheit einer organischen Base.

Das erfindungsgemäße Verfahren führt in einer überraschend kurzen Reaktionszeit zu den gewünschten Reaktionsprodukten bei gleichzeitig hoher Ausbeute.

Erfindungsgemäß erfolgt die Behandlung des Alkohols mit Ozon in Schritt b) in Anwesenheit einer anorganischen Base. Überraschenderweise kann eine sehr selektive Bildung des Aldehyds trotz der basischen Reaktionsbedingungen beobachtet werden, obwohl im allgemeinen Aldehyde unter basischen Reaktionsbedingen zur Bildung der entsprechenden Aldolkondensationsprodukte neigen.

Ferner war die Erreichbarkeit hoher Ausbeuten bei gleichzeitig kurzer Reaktionszeit insbesondere deshalb überraschend, da bekannt war, dass Ozon in Anwesenheit einer anorganischen Base (insbesondere bei schwächer basischen pH-Werten) rasch zerfällt (Hollemann, Wiberg, Lehrbuch der Anorganischen Chemie, W. de Gruyter 1995, 101. Aufl., S. 516). Dementsprechend war zu erwarten, dass große Mengen an Ozon benötigt würden, um eine ausreichende Menge an Ozon zum Umsetzen des sekundären Allylalkohols bereitzustellen. Es hat sich nunmehr überraschenderweise herausgestellt, dass die Menge an benötigtem Ozon trotz der Anwesenheit einer anorganischen Base im Vergleich zu herkömmlichen Verfahren nicht erhöht ist, und dass die erfindungsgemäße, vorzugsweise in einem Schritt durchgeführte Reaktion in Anwesenheit einer anorganischen Base sogar deutlich schneller und mit geringerem Bedarf an Sicherheitsmaßnahmen als bei herkömmlichen Verfahren durchführbar ist. Ebenfalls konnte gezeigt werden, dass durch die Umsetzung des sekundären Allylalkohols mit Ozon unter den genannten Bedingungen keine bzw. keine nennenswerte Weiteroxidation zur entsprechenden Carbonsäure zu beobachten ist. Dies kann unter den Bedingungen der Oxidation mit metallhaltigen, anorganischen Verbindungen nur teilweise erzielt werden.

Durch diese Variante der Ozonolyse ist ferner die Möglichkeit eröffnet worden, Moleküle mit Formylgruppen aus einem Halogenid und Acrolein (Propenal) zu generieren, wobei durch diese Reaktionssequenz das Ausgangsmolekül um ein Kohlenstoffatom verlängert worden ist, wobei R1 auch nachfolgend die eingangs angegebene Bedeutung besitzt:

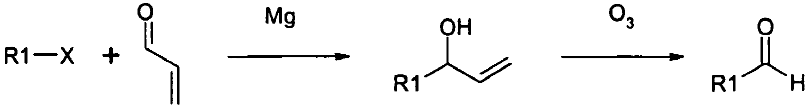

Dies stellt neben der Hydroformylierung somit eine vielseitigere Methode dar als die bisher verwendeten Methoden in der Literatur. Als Halogenide R1-X sind Chloride, Bromide und Iodide möglich. Die Addition des Kohlenstoffrestes an das Propenal erfolgt hierbei unter den herkömmlichen, aus der Literatur bekannten Methoden (Organikum, 16. Aufl., S. 495ff, VEB Deutscher Verlag der Wiisenschaften. 1985).

Es ist ebenfalls möglich, durch eine Reaktionssequenz, bestehend aus der nukleophilen Addition einer Vinylgruppe (beispielsweise mittels Vinyllithium oder eines Vinylmagnesiumhalogenids) an einen Aldehyd und durch anschließende Ozonolyse eine technisch nutzbare Methode für die Einführung und Entfernung von Schutzgruppen bereitzustellen:

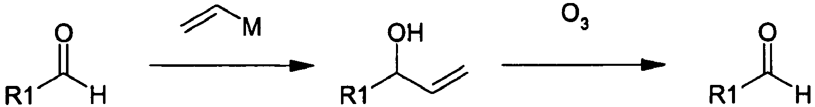

M bedeutet vorzugsweise MgCl, MgBr, MgI oder Li.

Ein weiterer Vorteil des erfindungsgemäßen Verfahrens ist, dass bei der Durchführung in Anwesenheit einer anorganischen Base während des Schrittes b) nur geringe Wärmemengen freigesetzt werden. Im Vergleich zu herkömmlichen Verfahren werden deshalb zum Durchführen des erfindungsgemäßen Verfahrens nur noch Kühlaggregate mit geringerer Leistung benötigt. Dies ist insbesondere bei einer industriellen Verfahrensführung ein großer Vorteil.

Besonders gute Ergebnisse können erhalten werden, wenn in Schritt b) der eingesetzte Alkohol mit 1-3 Moläquivalenten (molaren Äquivalenten) Ozon, bezogen auf die umzusetzende Alkohol-Gruppe, behandelt wird. Besonders bevorzugt ist dabei ein erfindungsgemäßes Verfahren, bei dem in Schritt b) der eingesetzte Alkohol mit 1-2 Moläquivalenten Ozon, bezogen auf die umzusetzende Alkohol-Gruppe, behandelt wird. In beiden Fällen wird die Menge an eingesetztem Ozon vorteilhaft gering gehalten. Dies ist insbesondere bei einer industriellen Verfahrensdurchführung von Vorteil, da Ozon zweckmäßigerweise während der Umsetzung des Alkohols in einer parallel laufenden Reaktion erzeugt und fortgesetzt oder kontinuierlich der in Schritt b) ablaufenden Reaktion zugesetzt wird. Das erfindungsgemäße Verfahren ermöglicht daher eine Umsetzung des Alkohols bei einem geringen Bedarf an bereitzustellendem Ozon.

Zur Erzeugung des Ozons in einem Ozongenerator können reiner Sauerstoff aber auch Gemische aus Sauerstoff und inerten Gasen in verschiedenen Volumen-Verhältnissen an Sauerstoff, vorzugsweise zwischen 1 und 80 Vol.%, verwendet werden. Ein Ozongehalt eines in Schritt b) in das Reaktionsgemisch eingeleiteten Gases liegt vorzugsweise im Bereich von 1 bis 12 Gew.% bezogen auf das eingesetzte Gas, besonders bevorzugt aber im Bereich von 4 bis 8 Gew.%. Das Ozon kann in einer molaren Menge im Bereich von 1 bis 5, bevorzugt im Bereich von 1 bis 3, besonders bevorzugt im Bereich von 1,1 bis 2 molaren Äquivalenten zur umzusetzenden alpha-ständigen Doppelbindung der Verbindung in das Reaktionsgemisch eingeleitet werden. Hierdurch lassen sich Nebenprodukte der Ozonolyse mindern.

Ferner ist es bevorzugt, wenn in Schritt b) die Base nicht bereits zu Beginn der Reaktion vollständig vorgelegt wird, sondern so fortgesetzt zugesetzt wird, dass ihre Äquivalentkonzentration bei Abbruch der Reaktion bezogen auf die insgesamt eingesetzten, umzusetzenden Alkohol-Gruppen 1 bis 3, vorzugsweise 1 bis 2 beträgt. Auf diese Weise ist sichergestellt, dass die Konzentration an verfügbarem Ozon optimal hoch ist, um eine rasche Umsetzung des Alkohols unter hohen Ausbeuten zu erzielen, und gleichzeitig gering genug ist, um das Freisetzen hoher Wärmemengen in Schritt b) zu verhindern. Die Base kann bei Durchführen des Schrittes b) kontinuierlich oder wiederholt zugesetzt werden.

Als anorganische Basen eignen sich alle unter Ozonolysebedingungen stabilen starken bis mittelstarken Brönstedtbasen. Die in Schritt b) verwendete Base ist vorzugsweise ausgewählt aus der Gruppe bestehend aus NaOH, KOH, LiOH, NaHCO₃, Na₂CO₃, CaCO₃ oder Mischungen zweier oder mehrerer dieser Basen. Vorteilhaft wiederum sind dabei die genannten Alkalimetallbasen, bevorzugt sind die Alkalimetallhydroxide. Besonders bevorzugte Basen sind NaOH, KOH und LiOH, am meisten bevorzugt sind NaOH und KOH. Mit Alkalimetallbasen werden in dem erfindungsgemäßen Verfahren keine entsprechenden Alkalimetallperoxide oder Alkalimetallozonide gebildet bzw. akkumuliert, im Gegensatz zu dem in US 3,664,810 beschriebenen Verfahren mit Erdalkalimetallbasen, bei dem im Wesentlichen stöchiometrische Mengen der entsprechenden Erdalkaliperoxide entstehen. Diese Basen, insbesondere die (besonders bevorzugt genannten) Alkalimetallhydroxide, haben in Vergleichsversuchen besonders hohe Ausbeuten der gewünschten Verbindung geliefert. Die Basen werden in Schritt b) zweckmäßigerweise in gelöster Form bereitgestellt, so dass bei der Auswahl der Base auch deren Löslichkeit in dem verwendeten Lösungsmittel zu berücksichtigen ist.

EP 1 569 885 betrifft die in-situ Zersetzung von Peroxiden bei der Ozonolyse von gegebenenfalls substituierten Alkenen zu den korrespondierenden Aldehyden oder Ketonen. Als Trägermaterial für die dort verwendeten Peroxid-zersetzenden Metallkatalysatoren kann unter anderem CaCO₃ verwendet werden. Gemäß EP 1 569 885 wird CaCO₃ nicht wegen seiner basischen Eigenschaften eingesetzt, sondern im Gegenteil als inertes Trägermaterial, welches sich in dem bei der Ozonolyse eingesetzten Lösungsmittel(gemisch) nicht auflöst und daher auch keinen nennenswerten Beitrag zum pH-Wert liefert.

Vorzugsweise wird das erfindungsgemäße Verfahren bei einem pH-Wert im Bereich von 13 bis 8 durchgeführt. Regelmäßig liegt der pH-Wert während der Ozonolyse in Anwesenheit der Base in Schritt b) des erfindungsgemäßen Verfahrens anfangs im Bereich von 13 bis 12, zum Ende der Ozonolyse im Bereich von 9 bis 8. Dabei ist anzumerken, dass die Selektivität der Ozonolyse in dem erfindungsgemäßen Verfahren mit fortschreitender Reaktionsdauer nicht abnimmt. Dies ist umso überraschender als dass bekannt ist, dass die Stabilität des Ozons bei niedrigeren pH-Werten (unterhalb von 14) merklich abnimmt (Hollemann, Wiberg, Lehrbuch der Anorganischen Chemie, W. de Gruyter 1995, 101. Aufl., S. 508, 517). Es wäre zu erwarten gewesen, dass die Zerfallsprodukte des Ozons mit den erfindungsgemäß einzusetzenden tertiären Alkoholen mit einer alpha-ständigen Doppelbindung zu unerwünschten Neben- oder Abbauprodukten der tertiären Alkohole reagiert hätten.

In bevorzugten Ausgestaltungen wird das erfindungsgemäße Verfahren in Abwesenheit eines heterogenen, anorganischen Peroxid-zersetzenden Katalysators aus der Gruppe Iridium, Mangan, Kobalt, Silber, Gold, Palladium, Platin oder Ruthenium durchgeführt.

In weiteren bevorzugten Ausgestaltungen wird das erfindungsgemäße Verfahren in Abwesenheit eines Emulgators durchgeführt.

Vorzugsweise wird als Lösungsmittel für die Base in Schritt b) Wasser oder ein Lösungsmittelgemisch aus Wasser und einem mit Wasser mischbaren organischen Lösungsmittel eingesetzt. Das Lösungsmittelgemisch besteht vorzugsweise aus Tetrahydrofuran und Wasser, insbesondere mit einem Massenmischungsverhältnis von Tetrahydrofuran zu Wasser im Bereich von 1:2 bis 2:1, besonders bevorzugt etwa 1:1. Das Lösungsmittel muss für die Ozonolyse geeignet sein. Die Base wird vorzugsweise in Schritt b) durch Zutropfen aus einer Stammlösung zugesetzt, wobei die Konzentration der Base in der Stammlösung vorzugsweise 2 bis 50 Gew.%, besonders bevorzugt 7,5 bis 10 Gew.% beträgt, jeweils bezogen auf die gesamte Stammlösung.

Das Lösungsmittel für den Alkohol ist so ausgewählt, dass es sich gegenüber Ozon vollständig oder weitgehend inert verhält und gegenüber der zugesetzten Base vollständig oder weitgehend stabil ist. Bevorzugte Lösungsmittel für den Alkohol umfassen substituierte oder nicht-substituierte aromatische Kohlenwasserstoffe, oder Lösungsmittel, die Sauerstoff in Form von Carbonyl-, Ether- oder Alkoholfunktionalitäten besitzen. Halogenierte aromatische und nichtaromatische Lösungsmittel erweisen sich ebenfalls als geeignet für die Durchführung der Reaktion. Lösungsmittel mit anderen oxidierbaren Heteroatomen (Stickstoff und Schwefel) sind aufgrund ihrer Affinität zu Sauerstoff wenig oder nicht geeignet. Besonders bevorzugt ist Toluol.

Insbesondere ist bevorzugt, dass die Reaktion in Schritt b) in einem Zweiphasensystem durchgeführt wird, wobei in Schritt a) der Alkohol in einem organischen Lösungsmittel bereitgestellt wird und in Schritt b) die Base in einem wässrigen Lösungsmittel eingesetzt wird. Dies hat den Vorteil, dass eine Ausfällung der Base bei der Zugabe in das Reaktionsgemisch verhindert wird, eine Konzentration der Base in der den Alkohol enthaltenden Phase des Reaktionsgemisches gering bleibt und eine Reaktion zwischen dem gebildeten Ozonid und der Base nur im Bereich der Phasengrenzen erfolgt. Vorteilhaft ist ebenfalls die Zurückdrängung von Parallel- und Konsekutivreaktionen (z.B. Aldolreaktion) durch die Verwendung eines Mehrphasensystems. Zweckmäßigerweise wird dabei das Reaktionsgemisch durch Rühren durchmischt. Besonders bevorzugt ist das Lösungsmittel des Alkohols Toluol und das Lösungsmittel der Base Wasser oder ein Lösungsmittelgemisch aus Wasser und Tetrahydrofuran, insbesondere mit einem Massenmischungsverhältnis von Tetrahydrofuran zu Wasser im Bereich von 1:2 bis 2:1, besonders bevorzugt etwa 1:1.

Vorzugsweise wird die Base in Schritt b) dem Reaktionsgemisch mit einer Zugaberate in Abhängigkeit von der Menge des gebildeten Ozonids zugesetzt. In der Regel wird die Zugaberate der Base erhöht, wenn sich auch die gebildete Menge des Ozonids im betrachteten Zeitraum erhöht, und umgekehrt. Besonders bevorzugt ist, wenn die gelöste Base in einer molaren Menge zwischen 0,8 bis 1,2 Mol-Äquivalenten zum gebildeten Ozonid zugesetzt wird. Hierdurch kann erreicht werden, dass die Konzentration des Ozonids im Reaktionsgemisch gering gehalten wird, aber mögliche Nebenreaktionen oder Störungen bei der Bildung des Ozonids aufgrund der zugesetzten Base vermieden werden.

Vorzugsweise beträgt in Schritt b) die Reaktionstemperatur -78 °C bis +30 °C, insbesondere -30°C bis +10°C, besonders bevorzugt -10°C bis 0°C. Hierdurch können Nebenreaktionen der Ozonolyse und bei der weiteren Umsetzung des gebildeten Ozonids mit der Base unterdrückt werden, aber gleichzeitig noch hinreichend hohe Umsätze für die beiden Teilreaktionen aufrecht erhalten werden.

Der in einem erfindungsgemäßen Verfahren, insbesondere nach einer der oben beschriebenen bevorzugten Verfahrensgestaltungen, eingesetzte Allylalkohol besitzt vorzugsweise die allgemeine Formel (la) 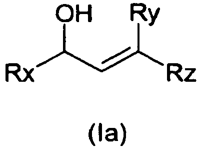 wobei Rx einen organischen Rest bedeutet, und wobei ferner Ry und Rz, unabhängig voneinander, Wasserstoff oder substituiertes oder nicht substituiertes Alkyl, Alkenyl, Cycloalkyl oder Aryl bedeuten können und beide Reste gemeinsam einen Ring bilden können und/oder einer oder beide Reste Ry und Rz mit dem Rest Rx gemeinsam einen Ring bilden können.

Besonders bevorzugt ist es, wenn Rx einen organischen Rest, vorzugsweise einen organischen Rest mit höchstens 50 Kohlenstoffatomen, bedeutet und

Ry und Rz, unabhängig voneinander, Wasserstoff oder substituiertes oder nicht substituiertes Alkyl, Alkenyl, Cycloalkyl, Heterocycloalkyl, Cycloalkylalkylen oder (Hetero)Aryl bedeuten können,
oder
Ry und Rz gemeinsam einen Ring, vorzugsweise einen insgesamt 5 bis 20 gliedrigen Ring, bilden können,
oder
einer oder beide Reste Ry und Rz mit dem Rest Rx gemeinsam einen Ring, vorzugsweise einen insgesamt 5 bis 20 gliedrigen Ring, bilden können.

Das erfindungsgemäße Verfahren ist mit einer Vielzahl strukturell sehr unterschiedlicher sekundärer Allylalkohole durchführbar.

Ry und Rz bedeuten unabhängig voneinander bevorzugt Wasserstoff oder substituiertes oder nicht substituiertes gerad- oder verzweigtkettiges C₁-C₂₀-Alkyl, gerad- oder verzweigtkettiges C₃-C₂₀-Alkenyl, C₃-C₂₀-Cycloalkyl, C₃-C₂₀-Heterocycloalkyl, C₃-C₂₀-Cycloalkylalkylen oder C₅-C₂₀-(Hetero)Aryl.

In bevorzugten Alkoholen der Formel (Ia) ist Rx ausgewählt aus organischen Resten mit bis zu 30 Kohlenstoffatomen und bis zu 10 Stickstoff- und/oder Sauerstoffatomen.

Besonders bevorzugt bedeutet Rx substituiertes oder nicht substituiertes gerad- oder verzweigtkettiges C₁-C₂₀-Alkyl, gerad- oder verzweigtkettiges C₃-C₂₀-Alkenyl, C₃-C₂₀-Cycloalkyl, C₃-C₂₀-Heterocycloalkyl, C₃-C₂₀-Cycloalkylalkylen oder C₅-C₂₀-(Hetero)Aryl.

Sofern es sich bei Rx, Ry und/oder Rz um eine substituiertes Alkyl, Alkenyl, Cycloalkyl, Heterocycloalkyl, Cycloalkylalkylen oder (Hetero)Aryl handelt, sind jeweils folgende Substituenten bevorzugt:
Hydroxy,
C₁-C₈-Alkyl, vorzugsweise Methyl, Ethyl, n-Propyl, iso-Propyl, n-Butyl, iso- Butyl, tert.- Butyl,
C₃-C₁₈-Cycloalkyl, vorzugsweise Cyclopropyl, Cyclopentyl, Cyclohexyl, Cyclooctyl, Cyclododecyl, Cyclopentadecyl, Cyclohexadecyl,
C₂-C₈-Alkinyl, vorzugsweise Ethinyl, Propinyl
C₁-Cₑ-Perfluoralkyl, vorzugsweise Trifluormethyl,
C₁-C₄-Alkoxy, vorzugsweise Methoxy, Ethoxy, iso-Propoxy, n-Butoxy, iso-Butoxy, tert.-Butoxy,
C₃-C₁₂-Cycloalkoxy, vorzugsweise C₃-Cycloalkoxy, C₅-Cycloalkoxy, C₆-Cycloalkoxy, C₈-Cycloalkoxy, C₁₂-Cycloalkoxy, C₁₅-Cycloalkoxy, C₁₆-Cycloalkoxy,
C₁-C₂₀-Alkoxyalkyl, in der 1 bis 5 CH₂-Gruppen durch Sauerstoff ersetzt sind, vorzugsweise -[-O-CH₂-CH₂-]ₙ-Q oder -[-O-CH₂-CHMe-]ₙ-Q, wobei Q = OH oder CH₃ ist und wobei n = 1 bis 4 bedeuten kann,
C₁-C₄-Acyl, vorzugsweise Acetyl,
C₁-C₄-Carboxy, vorzugsweise CO₂Me, CO₂Et, CO₂i-Pr, CO₂^{t}Bu,
C₁-C₄-Acyloxy, vorzugsweise Acetyloxy,
Halogenid, vorzugsweise F oder Cl, und
Si₁-Si₃₀-Siloxy.

Die guten Ergebnisse werden insbesondere dann erhalten, wenn die alphaständige Doppelbindung nicht Teil eines Systems konjugierter Doppelbindungen ist. Vorzugsweise sind die Reste Ry und Rz unabhängig voneinander deshalb Wasserstoff oder Alkyl, besonders bevorzugt bedeuten Ry und Rz Wasserstoff.

Wenn der umzusetzende Alkohol neben einer umzusetzenden Alkohol-Gruppe weitere Alkohol-Gruppen oder andere Gruppen trägt, die nicht umgesetzt werden sollen, dann werden diese Funktionalitäten zweckmäßigerweise gegen Ozonolyse geschützt.

Eine Derivatisierung der Verbindungen dient insbesondere der Einführung von Schutzgruppen für gegebenenfalls vorhandene nicht-allylische (nicht-alphaständige) Doppelbindungen, vorzugsweise durch deren selektive Epoxidierung.

Die Erfindung wird nachfolgend anhand von Ausführungsbeispielen näher erläutert.

### Allgemeine Angaben zur Reaktionsführung

Die Reaktionen wurden in herkömmlichen Laborapparaturen durchgeführt. In kleineren Ansätzen wurden die Reaktionsmischungen durch Trockeneisbäder bei der entsprechenden Temperatur gehalten. Bei größeren Ansätzen wurden Doppelmantelgefäße verwendet, durch die ein für den gewünschten Temperaturbereich geeignetes Kühlmedium gepumpt wurde.

In den Verbindungen vorhandene nicht-allylische Doppelbindungen wurden in der Regel vor der Ozonolyse durch Epoxidierung geschützt.

Soweit nicht anders angegeben, sind alle Angaben in % als Gewichts-%-Angaben zu verstehen. Mengenangaben in den Beispielen beziehen sich auf Massenverhältnisse. Raumtemperatur entspricht etwa 20°C.

### Beispiel 1 - Umsetzung von Oct-1-en-3-ol (VIII) mit Ozon zu Hexanal (IX)

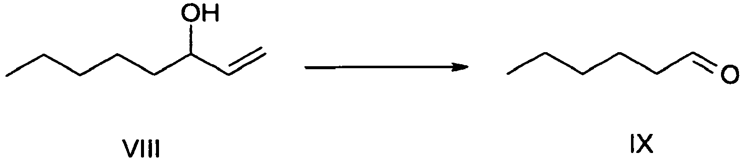

38,4 g (0,3 mol) Oct-1-en-3-ol werden in 218 g Toluol vorgelegt. Bei -5°C wird in die Reaktionsmischung ozonhaltiger Sauerstoff (32 g/h. 150 L, Gehalt an Ozon: 11 vol.-%) über 2 h eingeleitet. Über die gesamte Reaktionszeit wird in die Reaktionsmischung 24 g (0,6 mol) Natriumhydroxid in 456 g Wasser eingetropft. Nach Vertreiben des überschüssigen Ozons wird die Reaktionsmischung erwärmt und für 3 h bei Raumtemperatur gerührt. Die organische Phase wird anschließend abgetrennt, diese neutral und peroxidfrei gewaschen und zuletzt eingeengt. Umsatz Octenol: 90,8% (GC-MS)

Zur besseren Gehaltsbestimmung wird das gebildete Hexanal in toluolischer Lösung mit 24 g (0,31 mol) Propylenglykol mit 1 mol% para-Toluolsulfonsäure in das entsprechende Propylenglykolacetal umgesetzt. Durch GC-MS konnte die Bildung des Hexanalpropylenglykolacetals festgestellt werden.

Ausbeute Hexanalpropylenglykolacetal: 29,2 g, entsprechend 61,5% d.Theorie bezogen auf (IX)

### Beispiel 2 - Ozonolyse von 4-Methyltridec-1-en-3-ol (X) zu 2-Methylundecanal (XI)

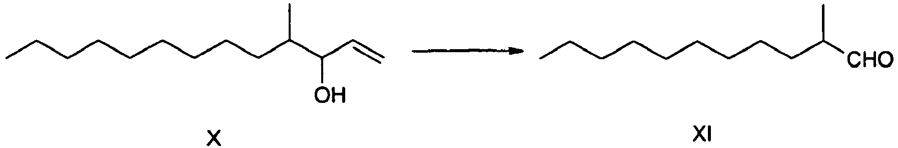

### Beispiel 2.1: Herstellung des sekundären Allylalkohols (X)

9,0 g (0,045 mol) 2-Methylundecanal werden in 100 g Tetrahydrofuran vorgelegt und die Lösung auf -25°C abgekühlt. In die Lösung wird unter Rühren bei dieser Temperatur Vinylmagnesiumbromidlösung (0,05 mol, 50 mL einer 1M Lösung in Tetrahydrofuran) eingetropft. Nach der Zugabe wird die Reaktionsmischung auf Raumtemperatur erwärmt und 2h unter Rückfluss erhitzt. Die Reaktionsmischung wird dann weitere 5 h bei Raumtemperatur gerührt und anschließend mit 20 g Wasser hydrolysiert. Nach abklingen der Wärmetönung wird der gebildete Niederschlag mit 5 %iger Salzsäure aufgelöst. Nach Abtrennung der wässrigen Phase wird die Reaktionsmischung mit 200 g Diethylether versetzt und zur Neutralisation mit 200 g Wasser gewaschen. Nach Abdestillieren des Lösemittels wird das Rohprodukt als schwach gelbes Öl erhalten.

Rohausbeute: 10,6 g, Gehalt: 90 % (GC-MS)

### Beispiel 2.2: Ozonolyse von (X) zu Methylundecanal (XI)

Das Produkt aus Beispiel 2.1 wird in 100 g Toluol aufgenommen und die Reaktionsmischung auf -5°C abgekühlt. Die Reaktionslösung wird dann über einen Zeitraum von 2 h mit ozonhaltigem Sauerstoff (10 vol.-% Ozon in Sauerstoff) begast. Während der gesamten Reaktionszeit werden zur Reaktionsmischung 1,5 Mol-Äquivalente an Natriumhydroxid bezogen auf den Allylalkohol, in Form einer 10 %igen Lösung in Wasser, zugetropft. Nach Ausgasen des unverbrauchten Ozons wird die organische Phase abgetrennt und neutral gewaschen. Nach Vernichtung eventuell noch vorhandener Peroxide wird durch Destillation im Vakuum das Lösungsmittel abgetrennt. Umsatz sek.
Allylalkohol: 100%
Rohausbeute: 9,67 g, Gehalt: 85,6% (GC-MS) entsprechend 80% d. Theorie bezogen auf (X)

### Beispiel 3 - Ozonolyse von 1-Phenylprop-2-enol (XII) zu Benzaldehyd (XIII)

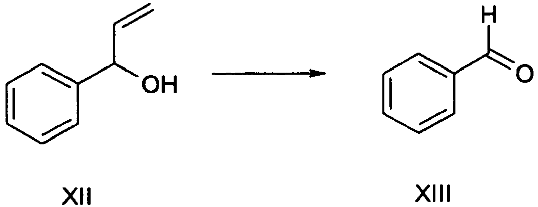

### Beispiel 3.1: Herstellung des Phenylprop-2-enols (XII)

5,0 g (0,05 mol) Benzaldehyd werden in 50 g Tetrahydrofuran gelöst und auf - 20°C abgekühlt. Zur dieser gekühlten Lösung wird das Vinylmagnesiumbromid als 1 molare Lösung in Tetrahydrofuran (1,05 molare Äquivalente bezogen auf den Benzaldehyd) zugetropft. Nach Zutropfen wird die Lösung auf Raumtemperatur erwärmt und für weitere 5 h bei Raumtemperatur gerührt. Die Reaktionslösung wird anschließend mit 100 g Wasser zur Hydrolyse versetzt. Nach Auflösen des Niederschlages mit 5 %iger Salzsäure wird die Reaktionslösung neutral gewaschen und die organische Phase im Vakuum vom Lösemittel befreit.

Rohausbeute: 6,0 g, Gehalt: 91% (GC-MS)

### Beispiel 3.2: Ozonolyse von Phenylprop-2-enol (XII) zu Benzaldehyd (XIII)

Der sekundäre Allylalkohol (XII) aus Beispiel 3.1 wird in 120 g Toluol gelöst und bei -5°C der Ozonolyse unterworfen. Während der gesamten Dauer der Ozonolyse wird eine äquimolare Menge (bezogen auf den sekundären Allylalkohol(XII)) an NaOH als 5%ige Lösung in Wasser zugetropft. Nach Ende der Ozonzugabe wird die Reaktionsmischung entgast und auf Raumtemperatur erwärmt. Nach Abtrennen der wässrigen Phase wird die organische Phase neutral und peroxidfrei gewaschen und die Reaktionsmischung im Vakuum vom Lösemitel befreit.
Rohausbeute: 3,88 g , Gehalt: 90% (GC-MS), entsprechend 80% d. Theorie

### Beispiel 4 - Ozonolyse von 1-Cyclohexylprop-2-enol (XIV) zu Cvclohexylcarbaldehyd (XV)

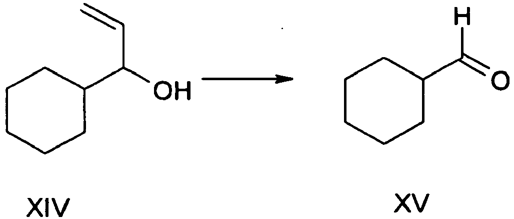

### Beispiel 4.1: Darstellung von Cyclohexylprop-2-enol (XIV) aus Acrolein und Cyclohexylbromid

2,4 g (0,1 mol) Mg-Späne werden in Diethylether (50 g) vorgelegt. Nach Aktivierung des Magnesiums mit Iod werden 16,3 g (0,1 mol) Cyclohexylbromid zugetropft, so dass die Reaktionsmischung gelinde siedet. Nach beendeter Zugabe wird die Mischung weitere 2h unter Rückfluss erwärmt, bis das Magnesium vollständig verbraucht ist. In die auf Raumtemperatur abgekühlte Lösung werden dann 5,9 g (0,11 mol) Acrolein zugetropft und zur Vervollständigung des Umsatzes die Reaktion weitere 1,5 h unter Rückfluss erhitzt. Die Aufarbeitung nach der Hydrolyse mit 100 g Wasser umfasst das Auflösen des Niederschlages, das Abtrennen der organischen Phase und die Neutralwäsche der organischen Phase, wie in Beispiel 3.1 beschrieben.
Rohausbeute: 10,7 g, Gehalt: 71%, (GC-MS)

### Beispiel 4.2: Ozonolyse von Cyclohexylpropen-2-ol (XIV) zu Cyclohexylcarbaldehyd (XV)

1,2 g des sekundären Allylalkohols (XIV) aus Beispiel 4.1 werden analog zu Beispiel 3.2 in der zwanzigfachen Masse an Toluol gelöst, bei -5°C der Ozonolyse unterworfen und aufgearbeitet.
Rohausbeute: 0,9 g
Umsatz sek. Allylalkohol: 100%, Selektivität Aldehyd: 37,9%

### Beispiel 5 - Darstellung von Nonanal (XVII) durch Ozonolyse von Undec-1-en-3-ol (XVI)

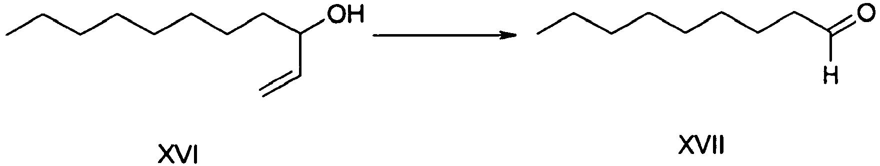

### Beispiel 5.1: Umsetzung von Octylbromid mit Acrolein zu (XVI)

Unter Einsatz von 19,3 g (0,10 mol) Octylbromid und den entsprechenden molaren Mengen an Magnesium und Acrolein gemäß Beispiel 4.1 erfolgt die Darstellung von Undec-1-en-3-ol (XVI).
Rohausbeute: 13,8 g, Gehalt: 80% (GC-MS)

### Beispiel 5.2: Ozonolyse zur Darstellung von Nonanal

Die Herstellung des Nonanals (XVII) aus dem sekundären Allylalkohol (XVI) des Beispiels 5.1 erfolgt analog zu Beispiel 3.2.
Nach Aufarbeitung wird Nonanal (XVII) als gelbliches Öl erhalten.

Umsatz sek. Allylalkohol: 24,3%, Selektivität Aldehyd: 100%

### Beispiel 6: Ozonolyse von 3,5,5-Trimethyl-cyclohex-2-enol (XVIII) zu 3,3-Dimethyl-5-oxo-hexanal (XIX)

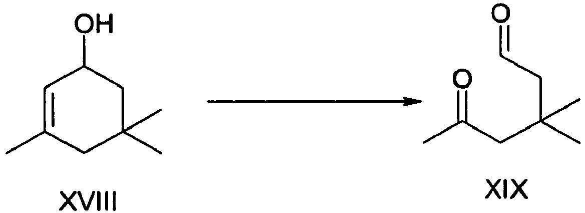

28 g (0,2 mol) des sekundären cyclischen Allylalkohols (XVIII) werden in 120 g Toluol gelöst und bei -10°C der Ozonolyse unterworfen. Während der gesamten Dauer der Ozonolyse (etwa 1,5 h) werden 1,5 molare Äquivalente bezogen auf den sekundären Allylalkohol (XVIII) an NaOH als 10%ige Lösung in Wasser zugetropft. Nach Ende der Ozonzugabe wird die Reaktionsmischung entgast und auf Raumtemperatur erwärmt. Nach Abtrennen der wässrigen Phase wird die organische Phase neutral- und peroxidfrei gewaschen und die Reaktionsmischung im Vakuum vom Lösemitel befreit.
Rohausbeute: 24,6 g (50% d.Theorie)
Umsatz Allylalkohol= 100%, Selektivität Aldehyd= 50% (GC-MS)

## Patentansprüche

1. Verfahren zur Darstellung eines Aldehyds aus einem sekundären Allylalkohol, umfassend die Schritte:
a) Bereitstellen des Alkohols,
b) Behandeln des Alkohols mit Ozon in Anwesenheit einer anorganischen Base.

2. Verfahren nach Anspruch 1, **dadurch gekennzeichnet, dass** in Schritt b) der eingesetzte Alkohol mit 1-3 Moläquivalenten Ozon je zu behandelnder Alkohol-Gruppe behandelt wird, und vorzugsweise mit 1-2 Moläquivalenten Ozon je zu behandelnder Alkohol-Gruppe behandelt wird.

3. Verfahren nach einem der vorherigen Ansprüche, **dadurch gekennzeichnet, dass** in Schritt b) eine anorganische Base derart zugesetzt wird, dass ihre Äquivalentkonzentration bezogen auf die zu behandelnde Alkohol-Gruppe 1 bis 3, vorzugsweise 1 bis 2 beträgt.

4. Verfahren nach Anspruch 3, **dadurch gekennzeichnet, dass** die Base in Schritt b) ausgewählt ist aus der Gruppe bestehend aus NaOH, KOH, LiOH, NaHCO₃, Na₂CO₃, CaCO₃ oder Mischungen zweier oder mehrerer dieser Basen.

5. Verfahren nach einem der Ansprüche 3 bis 4, **dadurch gekennzeichnet, dass** in Schritt b) die Base in einem wässrigen Lösungsmittel eingesetzt wird.

6. Verfahren nach einem der vorherigen Ansprüche, **dadurch gekennzeichnet, dass** in Schritt a) der Alkohol in einem organischen Lösungsmittel bereitgestellt wird.

7. Verfahren nach einem der vorherigen Ansprüche, **dadurch gekennzeichnet, dass** in Schritt b) die Reaktionstemperatur im Bereich von -78 °C bis +30 °C beträgt.

8. Verfahren nach einem der vorherigen Ansprüche, **dadurch gekennzeichnet, dass** der Alkohol die allgemeine Formel (Ia) besitzt: 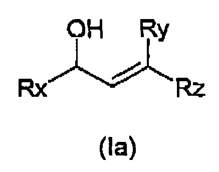 wobei Rx einen organischen Rest bedeutet, und wobei ferner Ry und Rz, unabhängig voneinander, Wasserstoff oder substituiertes oder nicht substituiertes Alkyl, Alkenyl, Cycloalkyl oder Aryl bedeuten können und beide Reste gemeinsam einen Ring bilden können und/oder einer oder beide Reste Ry und Rz mit dem Rest Rx gemeinsam einen Ring bilden können.

## Claims

1. Method for producing an aldehyde from a secondary allyl alcohol, comprising the steps:
a) providing the alcohol,
b) treating the alcohol with ozone in the absence of an inorganic base.

2. Method according to claim 1, **characterized in that** the alcohol used in step b) is treated with 1-3 mol equivalents ozone per each alcohol group to be treated, and preferably with 1-2 mol equivalents ozone per each alcohol group to be treated.

3. Method according to one of the previous claims, **characterized in that** an inorganic base is added in step b) so that its equivalent concentration with respect to the alcohol group to be treated is 1 to 3, preferably 1 to 2.

4. Method according to claim 3, **characterized in that** the base in step b) is selected from the group consisting of NaOH, KOH, LiOH, NaHCO₃, Na₂CO₃, CaCO₃ or mixtures of two or more of these bases.

5. Method according to one of claims 3 to 4, **characterized in that** the base in step b) is used in an aqueous solvent.

6. Method according to one of the previous claims, **characterized in that** the alcohol in step a) is provided in an organic solvent.

7. Method according to one of the previous claims, **characterized in that** the reaction temperature in step b) is within the range from -78 °C to +30 °C.

8. Method according to one of the previous claims, **characterized in that** the alcohol has the general formula (Ia): 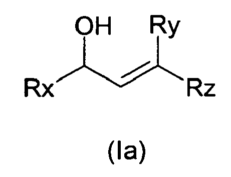 wherein Rx means an organic group, and wherein, furthermore, Ry and Rz, independently of each other can mean, hydrogen or substituted or non-substituted alkyl, alkenyl, cycloalkyl or aryl and both groups can form a ring together and/or one or both groups Ry and Rz can form a ring together with the group Rx.

## Revendications

1. Procédé de préparation d'un aldéhyde à partir d'un alcool allylique secondaire, comportant les étapes suivantes consistant à :
a) préparer l'alcool,
b) traiter l'alcool à l'ozone en présence d'une base inorganique.

2. Procédé selon la revendication 1, **caractérisé en ce qu'**à l'étape b), l'alcool utilisé est traité avec 1 à 3 équivalents molaires d'ozone pour former respectivement un groupe alcool à traiter, et de préférence avec 1 à 2 équivalents molaires d'ozone pour former le groupe alcool à traiter.

3. Procédé selon l'une quelconque des revendications précédentes, **caractérisées en ce qu'**à l'étape b), une base inorganique est ajoutée de telle manière que sa concentration équivalente est de 1 à 3, de préférence 1 à 2, rapportée au groupe alcool à traiter.

4. Procédé selon la revendication 3, **caractérisé en ce que** la base utilisée à l'étape b) est choisie dans le groupe constitué de NaOH, KOH, LiOH, NaHCO₃, Na₂CO₃, CaCO₃ ou de mélanges de deux ou plus de ces bases.

5. Procédé selon l'une quelconque des revendications 3 à 4, **caractérisé en ce qu'**à l'étape b), la base est placée dans un solvant aqueux.

6. Procédé selon l'une quelconque des revendications précédentes, **caractérisé en ce qu'**à l'étape a), l'alcool est préparé dans un solvant organique.

7. Procédé selon l'une quelconque des revendications précédentes, **caractérisé en ce qu'**à l'étape b), la température de réaction se situe dans la plage allant de -78 °C à +30 °C.

8. Procédé selon l'une quelconque des revendications précédentes, **caractérisé en ce que** l'alcool présente la formule générale (Ia) : 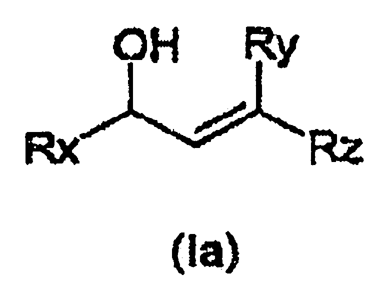 dans laquelle Rx représente un radical organique, et dans laquelle Ry et Rz peuvent en outre représenter, indépendamment l'un de l'autre, de l'hydrogène ou un alkyle, alcényle, cycloalkyle ou aryle substitué ou non substitué ou former conjointement entre eux un cycle, l'un ou les deux radicaux Ry et Rz pouvant former conjointement avec le radical Rx un cycle.
